# EUROPEAN PATENT SPECIFICATION

(11) **EP 1 434 620 B1**
(45) Date of publication and mention of the grant of the patent: **30.05.2007**
(21) Application number: 02800872.0
(22) Date of filing: 01.10.2002
(51) Int. Cl.: A61M 39/26, F16K 21/04, A61M 39/00

(54) **MALE LUER VALVE**
MÄNNLICHES LUER-VENTIL
SOUPAPE MALE LUER

(30) Priority: 09.10.2001 US 327817 P
(43) Date of publication of application: 07.07.2004
(60) Divisional application: 05026688.1
(73) Proprietor: HALKEY-ROBERTS CORPORATION, St. Petersburg, Florida 33716 (US)
(72) Inventor: ENERSON, Jon, R., Port Richey, FL 34668 (US)
(74) Representative: Long, Edward Anthony
(86) International application number: PCT/US2002/031346
(87) International publication number: WO 2003/030986

(56) References cited:
- EP-A- 0 365 300
- EP-A- 0 574 908
- EP-A- 0 639 389
- EP-A- 0 791 371
- EP-A- 1 108 443
- WO-A-00/59561
- WO-A-93/00956
- US-A- 1 939 128
- US-A- 2 503 495
- US-A- 3 199 831
- US-A- 3 396 743
- US-A- 4 089 504
- US-A- 4 378 028
- US-A- 4 436 125
- US-A- 5 135 025
- US-A- 5 277 402
- US-A- 5 429 155
- US-A- 5 950 986

## Description

### Background

This invention generally relates to valves such as luer lock valves which are used primarily in the medical field, and more specifically relates to a slidable type of valve used primarily in the medical field.

Slidable valves presently exist for use in the medical field. Such valves provide that the valve is initially biased into a closed position, where fluid cannot flow through the valve, and one or more internal components of the valve are slidable within the valve to actuate the valve into an open position, where fluid can flow through the valve.

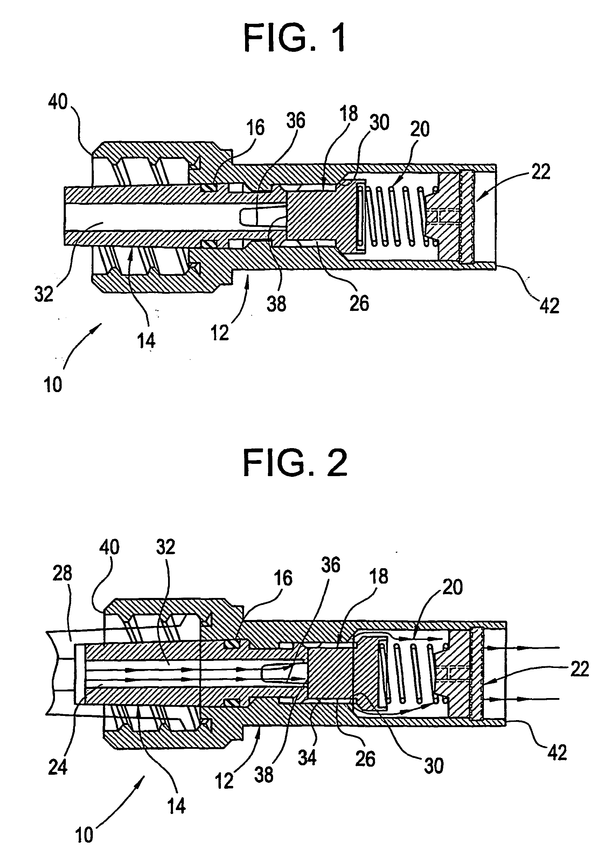

One type of medical valve is the subject of United States patent application Serial No. 09/523,354, and is shown in FIGURES 1 and 2 of the present application. Specifically, FIGURE 1 shows the valve 10 in the closed position (wherein fluid cannot flow through the valve), and FIGURE 2 shows the valve 10 in the open position (wherein fluid can flow through the valve). The valve 10 includes a valve body 12, a valve poppet 14 with luer taper (with sealing member 16 thereon), an internal resilient valve stem 18, a metal compression spring 20 and a valve plug 22, all of which are within the flow path of fluid moving through the valve (the arrows 24 shown in FIGURE 2 illustrate the fluid flow path (in one of two possible directions) through the valve 10). The valve stem 18 may include flutes or ribs on an external surface 26 thereof to facilitate fluid flow around the stem 18 when the valve 10 is in the open position.

In use, engagement or mating structure 28, such as a syringe, another valve or some other structure, engages the valve poppet 14, pushing it generally into the valve body 12 causing the valve 10 to move from the closed position as shown in FIGURE 1 to the open position as shown in FIGURE 2. As shown in FIGURE 2, when the valve 10 is in the open position, the valve stem 18 is disengaged from a valve seat 30 in the valve 10. This provides that fluid can ultimately flow from a bore 32 provided in the valve poppet 14 to an area 34 adjacent the periphery of the valve stem 18, or vice versa if the fluid is flowing in the opposite direction.

In the case where the fluid flows from left-to-right in FIGURE 2, fluid initially enters the bore 32 in the valve poppet 14 (i.e. from the mating structure 28), and travels to a notch 36 in the valve poppet 14 (and/or to a notch (not shown) in surface 38 of the valve stem 18). The valve stem 18 deflects the fluid to an area 34 adjacent the periphery of the valve stem 18, and the fluid flows along the external surface 26 of the valve stem 18 (and along the ribs, if provided, on the external surface 26 of the valve stem 18), past the valve seat 30, along the compression spring 20, and out the plug 22, and specifically between fins of the plug 22 and out the valve 10. In the opposite direction, fluid flows into the plug 22 of the valve 10, along the compression spring 20, past the valve seat 30, along the periphery of the valve stem 18 (and along the ribs, if provided, on the external surface 26 of the valve stem 18), to the notch 36 in the valve poppet 14 (and/or to a notch (not shown) in surface 38 of the valve stem 18), and through the bore 32 in the valve poppet 14 to the mating structure 28.

The overall design of the valve shown in FIGURES 1 and 2 -- being that there are so many components in the fluid flow path -- results in substantial restriction to fluid flow through the valve 10. As a result, the valve 10 cannot effectively conduct fluids having viscosities of 1.0 to 1.5 centipoise and above. Additionally, the design provides that there are numerous cavities or "dead areas" for entrapment of fluid within the valve 10. The existence of dead areas, and the fact that there so many components in the fluid flow path, creates turbulence in the fluid flow as the fluid flows through the valve 10. The turbulence renders the valve 10 a poor candidate for transmitting human blood, blood products, or any other material which is sensitive to turbulence. With regard to blood, concerns of lycing (i.e. damage to blood cells) and retention of clotted blood within the valve 10 gives rise to problems with possible infusion of thrombolotics or fibrous re-injection into a patient. The low viscosity conduction limits of the valve design shown in FIGURES 1 and 2 restrict,its utilization for high viscosity materials, thus limiting broader employment of the valve in a clinical environment.

Furthermore, the design shown in FIGURES 1 and 2 provides that while the valve poppet 14 is installed through the one end 40 of the valve 10, the other components (i.e. the valve stem 18, compression spring 20, and plug 22) are installed through the other end 42. This complicates and increases the cost of the assembly process.

In EP 0 574 908 is disclosed a valve comprising a valve body having a sealing surface and an annular internal slot, a valve core including ports and spring means engaged with the valve core, wherein the ports are aligned with the annular slot to allow fluid flow through the valve.

### Objects and Summary

A general object of an embodiment of the present invention is to provide a valve which has increased flow rate and an unobstructed fluid flow path.

Another object of an embodiment of the present invention is to provide a valve which has fewer components within the fluid flow path.

Still another object of an embodiment of the present invention is to provide a valve which causes less turbulence to the fluid flow.

Still yet another object of an embodiment of the present invention is to provide a valve which minimizes the residual volume (i.e. "dead areas") contributing to fluid entrapment.

Still yet another object of an embodiment of the present invention is to provide a method of assembling a valve wherein components are installed through one end of a valve body, but not the other.

In accordance with the present invention a valve is defined in Claim 1, with preferred or optional features being the subject of the appended sub-claims.

For instance, at least one end of the valve is preferably configured for a luer lock fitting.

A bore extends through the valve core, along a longitudinal axis thereof, and the bore defines a fluid flow area. The one or more ports on the valve core which align with the sealing surface of the valve body when the valve is in the closed position and with the one or more slots in the valve body when the valve is in the open position consists of one or more openings in a wall of the valve core. The valve body also includes a fluid flow area. Hence, a fluid flow path through the valve is defined by the fluid flow area defined through the valve core (i.e. the bore and the one or more ports) and the fluid flow area of the valve body. The spring means is generally between the valve body and valve core, but is not within the fluid flow path through the valve.

Preferably, each slot in the valve body is larger than each respective port of the valve core, and each port of the valve core is larger than a cross-sectional diameter of the bore which extends through the valve core. Preferably, the valve core includes two ports and the valve body includes two corresponding slots which align with each other when the valve core slides within the valve body to the open position. The ports of the valve core and the slots of the valve body are preferably 180 degrees apart relative to each other.

Preferably, a first sealing member and a second sealing member are disposed on the valve core, where the first sealing member engages the sealing surface of the valve body whether the valve core is in the open or the closed position, and the second sealing member engages with the sealing surface of the valve body when the valve core is in the closed position, but disengages from the sealing surface of the valve body when the valve core is in the open position. The valve core may include at least one barb which abuts against an internal surface of the valve body when the valve core is biased into the closed position by the spring means. Preferably, the valve body includes a pocket, the valve core includes a shoulder, and the spring means is disposed in the pocket of the valve body and engages the shoulder of the valve core Again, preferably the spring means is generally between the valve body and valve core, but is not within the fluid flow path through the valve. As an alternative to the sealing members, a resilient material may be over-molded or co-injected on the valve core to enhance the seal with the structure which is engaged with the valve and to enhance the seal between the valve core and valve body.

Another aspect of the present invention provides a method of assembling a valve. The method includes installing a plurality of components through one end of a valve body, and installing no components through an opposite end of the valve body. Hence, the assembly process is simplified and less costly.

### Brief Description of the Drawings

The organization and manner of the structure and operation of the invention, together with further objects and advantages thereof, may best be understood by reference to the following description, taken in connection with the accompanying drawings, wherein like reference numerals identify like elements in which:
FIGURE 1 is a side, cross-sectional view of the valve which is the subject of United States patent application Serial No. 09/523,354, showing the valve in a closed position;
FIGURE 2 is a view similar to FIGURE 1, but showing the valve in an open position;
FIGURE 3 is a side, cross-sectional view of a valve which is in accordance with an embodiment of the present invention, showing the valve in a closed position;
FIGURE 4 is a view similar to FIGURE 3, but showing the valve in an open position;
FIGURE 5 is an exploded view of the valve shown in FIGURES 3 and 4, illustrating that the valve is assembled from a distal end of the valve body; and
FIGURE 6 is a side, cross-sectional view of a valve which is in accordance with an another embodiment of the present invention.

### Description

While the present invention may be susceptible to embodiment in different forms, there are shown in the drawings, and herein will be described in detail, embodiments thereof with the understanding that the present description is to be considered an exemplification of the principles of the invention and is not intended to limit the invention to that as illustrated and described herein.

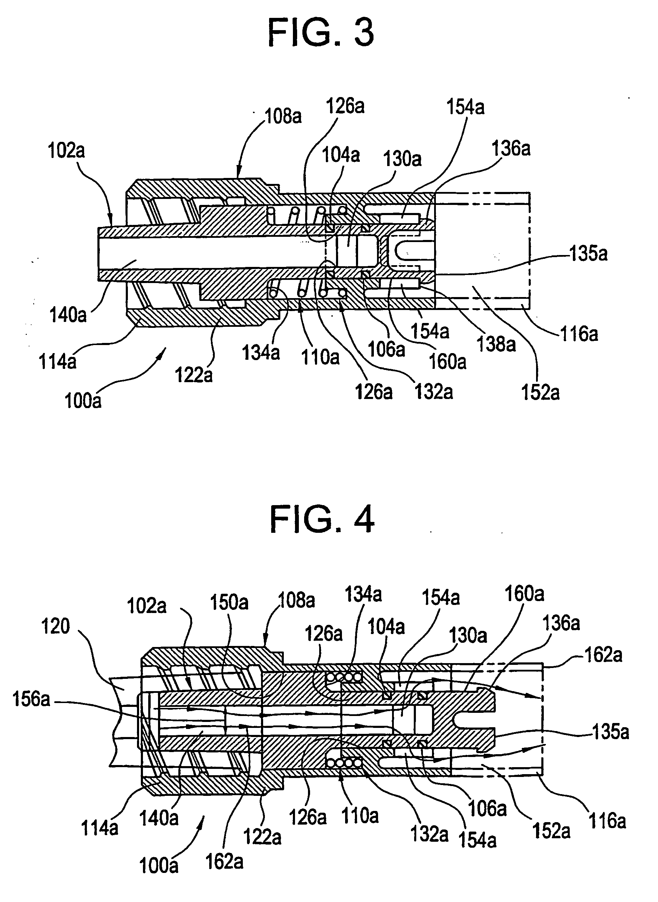

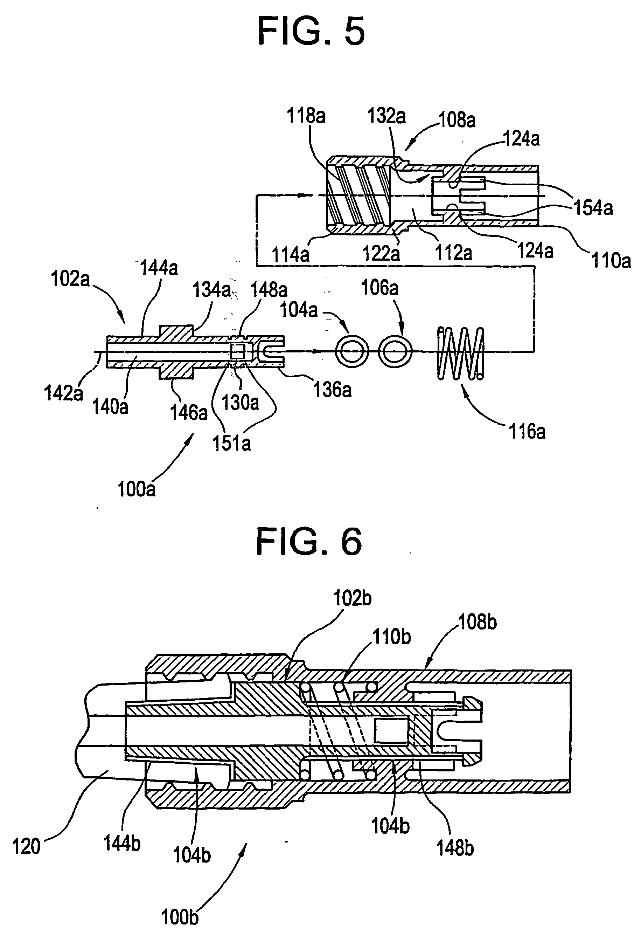

Several different valves are shown in FIGURES 3-9. A valve 100a which is in accordance with a first embodiment of the present invention is shown in FIGURES 3-5, a valve 100b which is in accordance with a second embodiment of the present invention is shown in FIGURE 6, a valve 100c which is in accordance with a third embodiment of the present invention is shown in FIGURE 7, a valve 100d which is in accordance with a fourth embodiment of the present invention is shown in FIGURE 8, and a valve 100e which is in accordance with a fifth embodiment of the present invention is shown in FIGURE 9.

Each of the valves shown in FIGURES 3-9 provides that fewer components are within the fluid flow path. As a result, each valve provides increased flow rate, a relatively unobstructed fluid flow path, and less turbulent fluid flow. Additionally, each valve minimizes the residual volume (i.e. "dead areas") contributing to fluid entrapment, and each provides enhanced backpressure tolerance when the valve is in the closed position. Additionally, the valves are inexpensive and easy to manufacture. Specifically, the valves shown in FIGURES 3-6 provide that assembly can be performed solely through one end of the valve body, as opposed to some components having to be installed through one end of the valve body and other components of the valve having to be installed through the other end of the valve body during the assembly process.

The valve 100a shown in FIGURES 3-5 will be described first and then the differences between the other valves 100b-100e and the valve 100a shown in FIGURES 3-5 will be described. The valve 100a shown in FIGURES 3-5 includes a valve core 102a, a pair of sealing members 104a, 106a which are disposed on the valve core 102a, a valve body 108a, and spring means 110a which is disposed in the valve body 108a, generally between the valve core 102a and the valve body 108a. The valve core 102a and valve body 108a are preferably made of plastic, while the sealing members 104a, 106a are preferably made of rubber or silicone, and the spring means 110a is preferably made of metal.

As shown, the valve body 108a is a generally hollow, cylindrical component having a central throughbore 112a. The valve body 108a may be made of, for example, clear plastic. The valve body 108a has a distal end 114a as well as a proximal end 116a which is generally opposite the distal end 114a. As shown in FIGURE 5, both the valve core 102a (including the sealing members 104a, 106a which are disposed thereon) and the spring means 110a are installed through the distal end 114a of the valve body 108a to assemble the valve 100a.

The distal end 114a of the valve body is preferably configured for a male luer fitting. Specifically, the valve body 108a preferably includes threading 118a at the distal end 114a for engagement with corresponding mating structure 120 (see FIGURE 4), such as a syringe, another valve, or some other structure, in a luer lock arrangement.

The valve body 108a includes a main body wall 122a which effectively defines the external surface of the valve 100a, and internal walls 124a which are connected to the main body wall 122a. As will be described more fully later herein, the internal walls 124a define sealing surfaces 126a which cooperate with ports 130a on the valve core 102a to prevent fluid flow through the valve 100a.

The internal walls 124a of the valve body 108a and the main body wall 122a of the valve body 108a define a pocket 132a, and the spring means 110a is disposed in the pocket 132a. Specifically, the spring means 110a is preferably a metal compression spring which has one end disposed in the pocket 132a in the valve body 108a and has an opposite end contactably engaged with a shoulder 134a on the valve core 102a. The end 135a of the valve core 102a preferably includes barbs 136a, or some other suitable structure, for generally retaining the valve core 102a in the valve body 108a, and preventing the valve core 102a from being pushed completely out of the valve body 108a by the compression spring 110a (via contactable engagement with internal surface 138a of the valve body 108a -- see FIGURE 3). Additionally, the barbs 136a provide that the valve core 102a can be snapped into the valve body 108a through the distal end 114a during assembly (see FIGURE 5).

The valve core 102a is a generally hollow, cylindrical member having a central throughbore 140a. The central throughbore 140a extends along a longitudinal axis 142a of the valve core 102a and defines a fluid flow area. The valve core 102a includes a forward portion 144a, middle portion 146a which provides shoulder 134a, and a rearward portion 148a. Consistent with the configuration of the distal end 114a of the valve body 108a, the forward portion 144a of the valve core 102a preferably has a male luer taper to facilitate the luer lock engagement with the corresponding mating structure. Specifically, preferably the forward portion 144a of the valve core 102a has a standard ANSI/ISO luer configuration. The middle portion 146a of the valve core 102a (which provides shoulder 134a) contactably engages an internal surface 150a of the valve body 108a and is disposed between the forward (144a) and rearward (148a) portions of the valve core 102a. As shown in FIGURE 4, when mating structure 120, such as a syringe, another valve, or some other structure is engaged with the valve 100a, the structure 120 pushably engages the middle portion 146a of the valve core 102a causing the valve core 102a to translate or slide relative to the valve body 108a which causes the compression spring 110a to compress and the valve 100a to actuate into the open position as shown in FIGURE 4.

As shown in FIGURES 3-5, the valve core 102a includes a pair of ports 130a on the rearward portion 148a of the valve core 102a. The ports 130a are effectively openings through the surface of the valve core 102a, in communication with the central throughbore 140a (and the fluid flow area defined thereby). Preferably, the ports 130a on the valve core 102a are 180 degrees apart relative to each other along the external circumferential surface of the rearward portion 148a of the valve core 102a.

The valve 100a shown in FIGURES 3-5 provides that there are a pair of sealing members 104a, 106a disposed on the valve core 102a proximate the ports 130a on the valve core 102a. The sealing members 104a, 106a may comprise o-rings. Specifically, one sealing member 104a is disposed on the valve core 102a between the ports 130a and the middle portion 146a of the valve core 102a, and another sealing member 106a is disposed on the valve core 102a between the ports 130a and the end 135a of the valve core 102a. As shown in FIGURE 5, glands 151a are preferably provided on the external surface of the valve core 102a for seating the sealing members 104a, 106a on the valve core 102a.

The valve 100a is configured such that the one sealing member 104a always remains engaged with the sealing surfaces 126a in the valve 100a which are provided by the internal walls 124a of the valve body 108a regardless of whether the valve 100a is in the closed (see FIGURE 3) or open (see FIGURE 4) position. Sealing member 104a prevents fluid from leaking into the area in which the spring 110a is located, and does so regardless of whether the valve 100a is in the closed (see FIGURE 3) or open (see FIGURE 4) position. The valve 100a is configured such that the other sealing member 106a is engaged with the sealing surfaces 126a when the valve 100a is in the closed position (see FIGURE 3), but becomes disengaged therefrom when the valve 100a is actuated into the open position (see FIGURE 4). Hence, the sealing member 106a prevents fluid communication between the valve ports 130a and a fluid flow area 152a of the valve body 108a when the valve 100a is in the closed (see FIGURE 3) position.

Additionally, the valve 100a is configured such that the ports 130a on the valve core 102a align with the sealing surfaces 126a in the valve 100a when the valve 100a is in the closed position (see FIGURE 3), but move out of alignment with the sealing surfaces 126a when the valve 100a is actuated into the open position (see FIGURE 4).

The valve body 108a includes internal slots 154a which correspond with the ports 130a in the valve core 102a, and fluid flow area 152a of the valve body 108a is adjacent the slots 154a. Preferably, each of the slots 154a in the valve body 108a is larger than each of the respective ports 130a of the valve core 102a, and each of the ports 130a of the valve core 102a is larger than a cross-sectional diameter 156a of the central throughbore 140a which extends through the valve core 102a. This limits the fluid flow only to that permitted by the inside diameter 156a of the standard ANSI/ISO luer configuration, which comprises the distal end 114a of the valve core 102a.

The valve core 102a also includes a seal surface 160a on its rearward portion 148a which effectively seals off the slots 154a on the valve body 108a when the valve 100a is in the closed position as shown in FIGURE 3. However, when the valve 100a is actuated into the open position (see FIGURE 4), the ports 130a of the valve core 102a move out of alignment with the sealing surfaces 126a of the valve 100a and into alignment with the corresponding slots 154a in the valve body 108a, thereby defining a fluid flow path through the valve 100a (one possible fluid flow direction is represented by arrows 162a shown in FIGURE 4). Specifically, in a direction from left-to-right in FIGURE 4, the fluid flow path is: into the throughbore 140a in the valve core 102a, through the ports 130a in the valve core 102a, through the corresponding slots 154a in the valve body 108a, into the fluid flow area 152a of the valve body 108a and out the end 162a of the valve 100a. In the other direction, i.e. in a direction from right-to-left in FIGURE 4, the fluid flow path is: into the end 162a of the valve 100a, through the slots 154a in the valve body 108a, through the ports 130a in the valve core 102a, and along (and out) the throughbore 140a in the valve core 102a.

Before mating structure 120, such as a syringe, another valve, or some other structure is engaged with the valve 100a, the valve 100a is in the closed position as shown in FIGURE 3. In the closed position, the valve core 102a is biased into the closed position by the spring means 110a, the ports 130a in the valve core 102a are aligned with the sealing surfaces 126a in the valve body 108a, and both sealing members 104a, 106a on the valve core 102a sealingly engage the sealing surfaces 126a, thereby preventing fluid flow between the central throughbore 140a in the valve core 102a and the fluid flow area 152a in the valve body 108a. When mating structure 120 engages the valve 100a, the mating structure 120 pushes the valve core 102a into the valve body 108a, causing the valve 100a to be actuated into the open position as shown in FIGURE 4. In the open position, the spring 110a is compressed, the ports 130a in the valve core 102a are aligned with the corresponding slots 154a in the valve body 108a, and only sealing member 104a on the valve core 102a remains sealingly engaged with the sealing surfaces 126a. Hence, fluid flow is permitted between the central throughbore 140a in the valve core 102a and the fluid flow area 152a in the valve body 108a.

The valve 100b shown in FIGURE 6 is very similar to that shown in FIGURES 3-5, and includes a valve core 102b, a valve body 108b and a spring means 110b very much like the valve 100a shown in FIGURES 3-5. However, instead of providing that two sealing members, such as o-rings, are disposed on the valve core, the valve 100b shown in FIGURE 6 provides that a sealing material 104b is co-injected or over-molded onto the exterior surface of the valve core 102b, on the forward 144b and rearward 148b portions. Functionally, the sealing material 104b which is on the rearward portion 148b of the valve core 100b performs the same function as the sealing members 104a, 106a shown in FIGURES 3-5. The sealing material 104b which is on the forward portion 144b of the valve core 102b works to provide a seal between the mating structure 120 and the valve core 102b. The sealing material 104b which is co-injected or over-molded onto the exterior surface of the valve core 102b may consist of a rigid substrate material with a different resilient outer surface material shell. The employment of co-injection or over-molding for the valve core 104b eliminates the requirement of o-ring seals in the design (see FIGURES 3-5) and further reduces the component part count.

Each of the valves 100a-100e shown in FIGURES 3-9 provides that fewer components are within the fluid flow path, that flow rate is increased (when the valve is open), that there is a relatively unobstructed fluid flow path, and that there is less turbulence introduced into the flow. Additionally, each valve minimizes dead areas which can contribute to fluid entrapment, and each provides enhanced backpressure tolerance when the valve is in the closed position. Additionally, the valves are inexpensive and easy to manufacture.

Specifically, valves 100a and 100b provide that assembly can be performed solely through one end (i.e. end 114a shown in FIGURE 5) of the valve body 108a, 108b, as opposed to some components having to be installed through one end of the valve body and other components of the valve having to be installed through the other end of the valve body during the assembly process.

While embodiments of the present invention are shown and described, it is envisioned that those skilled in the art may devise various modifications of the present invention without departing from the scope of the claims.

## Claims

1. A valve (100a) comprising: a valve body (108a) which includes a sealing surface (126a) and at least one internal slot (154a) extending through internal wall of the valve body (108a); and forming part of a fluid flow path through said valve (100a), a valve core (102a) disposed in said valve body and including at least one port (130a); spring means (110a) engaged with said valve body (108a) and said valve core (102a), said spring means (110a) biasing said valve core (102a) into a closed position wherein said port (130a) of said valve core (102a) is aligned with said sealing surface (126a) of said valve body (108a) thereby prohibiting fluid flow through said valve (100a) said valve core (102a) being slidable generally within said valve body (108a) to an open position wherein said port (130a) of said valve core (102a) is aligned with said internal slot (154a) of said valve body (108a) thereby allowing fluid flow through said internal fluid flow path form said valve core (102a), through said port (130a) and through said internal slot (154a) to an internal area of said valve body (108a), and out of an end of said valve (100a).

2. A valve (100a) as defined in claim 1, wherein said valve core (102a) includes a body and a bore (140a) through said body along a longitudinal axis (142a) of said body, wherein said body of said valve core (102a) includes a wall and said port (130a) comprises an opening in said wall.

3. A valve (100a) as defined in claim 2, wherein said valve body (108a) includes a fluid flow area and the bore (140a) through said body (108a) of said valve core (102a) defines a fluid flow area, wherein a fluid flow path through said valve (100a) is defined by said fluid flow area defined by said bore (140a) through said body (108a) of said valve core (102a) and said fluid flow area of said valve body (108a), and said spring means (110a) is not within said fluid flow path through said valve (100a)

4. A valve (100a) as defined in claim 2, wherein said slot (154a) in said valve body (108a) is larger than said port (130a) of said valve core (102a), and said port (130a) of said valve core (102a) is larger than a cross-sectional diameter of said bore (140a) through said valve core (102a).

5. A valve (100a) as defined in claim 1, wherein said valve core (102a) includes two ports (130a) and said valve body (108a) includes two slots (154a), said valve core (102a) being slidable generally within said valve body (108a) to an open position wherein said ports (130a) of said valve core (102a) are aligned with said slots (154a) of said valve body (108a) thereby allowing fluid flow through said valve (100a).

6. A valve (100a) as defined in claim 5, wherein said ports (130a) of said valve core (102a) are 180 degrees apart and said slots (154a) of said valve body (108a) are 180 degrees apart.

7. A valve (100a) as defined in claim 1, wherein said slot (154a) in said valve body (108a) is larger than said port (130a) of said valve core (102a).

8. A valve (100a) as defined in claim 1, wherein at least one end of said valve (100a) is configured for a Luer lock fitting.

9. A valve (100a) as defined in claim 1, further comprising at least one sealing member (104a, 106a) disposed on said valve core (102a) and engaged with said sealing surface (126a) of said valve body (108a).

10. A valve (100a) as defined in claim 1, further comprising a first sealing member (104a) disposed on said valve core (102a), said first sealing member (104a) engaged with said sealing surface (126a) of said valve body (108a) when said valve core (102a) is in said open and closed positions, and further comprising a second sealing member (106a) disposed on said valve core (102a), said second sealing member (106a) engaged with said sealing surface (126a) of said valve body (108a) when said valve core (102a) is in said closed position, but disengaged from said sealing surface (126a) of said valve body (108a) when said valve core (102a) is in said open position.

11. A valve (100a) as defined in claim 1, said valve core (102a) including at least one barb (136a) which abuts against an internal surface (138a) of said valve body (108a) when said valve core (102a) is biased into said closed position by said spring means (110a).

12. A valve (100a) as defined in claim 1, wherein said valve body (108a) includes a pocket (132a) and said valve core (102a) includes a shoulder (134a), wherein said spring means (110a) is disposed in said pocket (132a) of said valve body (108a) and engages said shoulder (134a) of said valve core (102a).

13. A valve (100a) as defined in claim 1, wherein a resilient material (104b) is over-molded or co-injected on said valve core (102b).

14. A valve (100a) as defined in claim 13, wherein said resilient material (104b) is disposed on said valve core (102a) proximate said port (130a).

## Patentansprüche

1. Ventil (100a) umfassend: ein Ventilgehäuse (108a) mit einer Dichtfläche (126a) und mindestens einem Innenschlitz (154a), der durch die Innenwand des Ventilgehäuses (108a) führt; und Teil eines Durchflusswegs durch das Ventil (100a) bildet; ein Ventileinsatz (102a), der im Ventilgehäuse angeordnet ist und mindestens eine Durchgangsöffnung (130a) umfasst; eine Feder (110a), die mit dem Ventilgehäuse (108a) und dem Ventileinsatz (102a) verbunden ist, wobei die Feder (110a) den Ventileinsatz (102a) in geschlossener Stellung vorspannt, wobei die Durchgangsöffnung (130a) des Ventileinsatzes (102a) mit der Dichtfläche (126a) des Ventilgehäuses (108a) ausgerichtet ist und damit den Fluss der Flüssigkeit durch das Ventil (100a) des Ventileinsatzes (102a) verhindert und dabei im Allgemeinen innerhalb des Ventilgehäuses (108a) in Richtung geöffnete Position verschiebbar ist, wobei die Durchgangsöffnung (130a) des Ventileinsatzes (102a) mit dem Innenschlitz (154a) des Ventilgehäuses (108a) ausgerichtet ist und damit der Flüssigkeit den Fluss durch den inneren Durchflussweg vom Ventileinsatz (102a) durch die Durchgangsöffnung (130a) und durch den Innenschlitz (154a) zum Innenteil des Ventilgehäuses (108a) und aus einem Ende des Ventils (100a) heraus ermöglicht.

2. Ventil (100a) nach Anspruch 1, wobei der Ventileinsatz (102a) ein Gehäuse und eine Bohrung (140a) durch das Gehäuse in Richtung Längsachse (142a) des Gehäuses umfasst, wobei das Gehäuse des Ventileinsatzes (102a) eine Wand aufweist und die Durchgangsöffnung (130a) eine Öffnung in der Wand umfasst.

3. Ventil (100a) nach Anspruch 2, wobei das Ventilgehäuse (108a) einen Durchflussbereich aufweist und die Bohrung (140a) durch das Gehäuse (108a) des Ventileinsatzes (102a) einen Durchflussbereich definiert, wobei der Durchflussweg durch das Ventil (100a) durch den Durchflussbereich definiert wird, der durch die Bohrung (140a) durch das Gehäuse (108a) des Ventileinsatzes (102a) definiert wird, und der Durchflussbereich des Ventilgehäuses (108a) und der Feder (110a) sich nicht innerhalb des Durchflusswegs durch das Ventil (100a) befindet.

4. Ventil (100a) nach Anspruch 2, wobei der Schlitz (154a) im Ventilgehäuse (108a) größer ist als die Durchgangsöffnung (130a) des Ventileinsatzes (102a) und die Durchgangsöffnung (130a) des Ventileinsatzes (102a) größer ist als der Querschnittsdurchmesser der Bohrung (140a) durch den Ventileinsatz (102a).

5. Ventil (100a) nach Anspruch 1, wobei der Ventileinsatz (102a) zwei Durchgangsöffnungen (103a) aufweist und das Ventilgehäuse (108a) zwei Schlitze (154a) aufweist, wobei der Ventileinsatz (102a) im Allgemeinen innerhalb des Ventilgehäuses (108a) in eine geöffnete Position verschiebbar ist, wobei die Durchgangsöffnungen (130a) des Ventileinsatzes (102a) mit den Schlitzen (154a) des Ventilgehäuses (108a) ausgerichtet sind und damit einen Durchfluss durch das Ventil (100a) ermöglichen.

6. Ventil (100a) nach Anspruch 5, wobei die Durchgangsöffnungen (130a) des Ventileinsatzes (102a) um 180° voneinander versetzt sind und die Schlitze (154a) des Ventilgehäuses (108a) 180° voneinander versetzt sind.

7. Ventil (100a) nach Anspruch 1, wobei der Schlitz (154a) im Ventilgehäuse (108a) größer ist als die Durchgangsöffnung (130a) des Ventileinsatzes (102a).

8. Ventil (100a) nach Anspruch 1, wobei mindestens ein Ende des Ventils (100a) für eine Luer-Lock-Verbindung konfiguriert ist.

9. Ventil (100a) nach Anspruch 1, weiterhin umfassend mindestens ein Dichtungsteil (104a, 106a), das am Ventileinsatz (102a) angeordnet ist und mit der Dichtfläche (126a) des Ventilgehäuses (108a) in Verbindung steht.

10. Ventil (100a) nach Anspruch 1, weiterhin umfassend ein erstes Dichtungsteil (104a), das am Ventileinsatz (102a) angeordnet ist, wobei das erste Dichtungsteil (104a) mit der Dichtfläche (126a) des Ventilgehäuses (108a) in Verbindung ist, sobald der Ventileinsatz (102a) sich in geöffneter oder geschlossener Position befindet, weiterhin umfassend ein zweites Dichtungsteil (106a), das am Ventileinsatz (102a) angeordnet ist, wobei das zweite Dichtungsteil (106a) mit der Dichtfläche (126a) des Ventilgehäuses (108a) in Verbindung ist, sobald der Ventileinsatz (102a) sich in geschlossener Position befindet, aber nicht mit der Dichtfläche (126a) des Ventilgehäuses (108a) in Verbindung steht, sobald der Ventileinsatz (102a) sich in geöffneter Position befindet.

11. Ventil (100a) nach Anspruch 1, wobei der Ventileinsatz (102a) mindestens einen Widerhaken (136a) aufweist, der an der Innenfläche (138a) des Ventilgehäuses (108a) anliegt, sobald der Ventileinsatz (102a) durch die Feder (110a) in die geschlossene Position gedrückt wird.

12. Ventil (100a) nach Anspruch 1, wobei das Ventilgehäuse (108a) eine Tasche (132a) aufweist und der Ventileinsatz (102a) einen Absatz (134a) aufweist, wobei die Feder (110a) sich in der Tasche (132a) des Ventilgehäuses (108a) befindet und mit dem Absatz (134a) des Ventileinsatzes (102a) in Verbindung steht.

13. Ventil (100a) nach Anspruch 1, wobei der Ventileinsatz (102b) mit dem flexiblen Material (104b) überzogen oder überspritzt wurde.

14. Ventil (100a) nach Anspruch 13, wobei das flexible Material (104b) in der Nähe der Durchgangsöffnung (130a) am Ventileinsatz (102a) angeordnet ist.

## Revendications

1. Soupape (100a) comprenant: un corps de soupape (108a) qui comprend une surface d'étanchéité (126a) et au moins une fente interne (154a) s'étendant au travers d'une paroi interne du corps de soupape (108a) ; et faisant partie d'un trajet d'écoulement de fluide à travers ladite soupape (100a), une pièce intérieure de soupape (102a) disposée dans ledit corps de soupape et comprenant au moins un orifice (130a) ; des moyens de ressort (110a) en prise avec ledit corps de soupape (108a) et ladite pièce intérieure de soupape (102a), lesdits moyens de ressort (110a) sollicitant ladite pièce intérieure de soupape (102a) dans une position fermée dans laquelle ledit orifice (130a) de ladite pièce intérieure de soupape (102a) est aligné avec ladite surface d'étanchéité (126a) dudit corps de soupape (108a) empêchant de ce fait un écoulement de fluide à travers ladite soupape (100a), ladite pièce intérieure de soupape (102a) étant susceptible de coulisser d'une façon générale à l'intérieur dudit corps de soupape (108a) vers une position ouverte dans laquelle ledit orifice (130a) de ladite pièce intérieure de soupape (102a) est aligné avec ladite fente interne (154a) dudit corps de soupape (108a) rendant ainsi possible un écoulement de fluide à travers ladite soupape, depuis ladite pièce intérieure de soupape (102a), en passant à travers ledit orifice (130a) et à travers ladite fente interne (154a), jusqu'à une région interne dudit corps de soupape (108a), et en sortie hors d'une extrémité de ladite soupape (100a).

2. Soupape (100a) selon la revendication 1, dans laquelle ladite pièce intérieure de soupape (102a) comprend un corps, et un alésage (140a) à travers ledit corps le long d'un axe longitudinal (142a) dudit corps, dans laquelle ledit corps de ladite pièce intérieure de soupape (102a) comprend une paroi, et ledit orifice (130a) comprend une ouverture dans ladite paroi.

3. Soupape (100a) selon la revendication 2, dans laquelle ledit corps de soupape (108a) comprend une région d'écoulement de fluide, et l'alésage (140a) à travers ledit corps (108a) de ladite pièce intérieure de soupape (102a) définit une région d'écoulement de fluide, dans laquelle un trajet d'écoulement de fluide à travers ladite soupape (100a) est défini par ladite région d'écoulement de fluide définie par ledit alésage (140a) à travers ledit corps (108a) de ladite pièce intérieure de soupape (102a) et ladite région d'écoulement de fluide dudit corps de soupape (108a), et lesdits moyens de ressort (110a) ne se trouvent pas sur ledit trajet d'écoulement de fluide à travers ladite soupape (100a)

4. Soupape (100a) selon la revendication 2, dans laquelle ladite fente (154a) dans ledit corps de soupape (108a) est plus large que ledit orifice (130a) de ladite pièce intérieure de soupape (102a), et ledit orifice (130a) de ladite pièce intérieure de soupape (102a) est plus large qu'un diamètre en coupe transversale dudit alésage (140a) à travers ladite pièce intérieure de soupape (102a).

5. Soupape (100a) selon la revendication 1, dans laquelle ladite pièce intérieure de soupape (102a) comprend deux orifices (130a) et ledit corps de soupape (108a) comprend deux fentes (154a), ladite pièce intérieure de soupape (102a) étant susceptible de coulisser d'une façon générale à l'intérieur dudit corps de soupape (108a) vers une position ouverte dans laquelle lesdits orifices (130a) de ladite pièce intérieure de soupape (102a) sont alignés avec lesdites fentes (154a) dudit corps de soupape (108a), rendant ainsi possible un écoulement de fluide à travers ladite soupape (100a).

6. Soupape (100a) selon la revendication 5, dans laquelle lesdits orifices (130a) de ladite pièce intérieure de soupape (102a) se trouvent à 180 degrés l'un par rapport à l'autre, et lesdites fentes (154a) dudit corps de soupape (108a) se trouvent à 180 degrés l'une par rapport à l'autre.

7. Soupape (100a) selon la revendication 1, dans laquelle ladite fente (154a) dans ledit corps de soupape (108a) est plus large que ledit orifice (130a) de ladite pièce intérieure de soupape (102a).

8. Soupape (100a) selon la revendication 1, dans laquelle au moins une extrémité de ladite soupape (100a) est configurée pour une pièce de fixation Luer.

9. Soupape (100a) selon la revendication 1, comprenant en outre au moins un élément d'étanchéité (104a, 106a) disposé sur ladite pièce intérieure de soupape (102a) et en prise avec ladite surface d'étanchéité (126a) dudit corps de soupape (108a).

10. Soupape (100a) selon la revendication 1, comprenant en outre un premier élément d'étanchéité (104a) disposé sur ladite pièce intérieure de soupape (102a), ledit premier élément d'étanchéité (104a) étant en prise avec ladite surface d'étanchéité (126a) dudit corps de soupape (108a) quand ladite pièce intérieure de soupape (102a) se trouve dans lesdites positions ouverte et fermée, et comprenant en outre un deuxième élément d'étanchéité (106a) disposé sur ladite pièce intérieure de soupape (102a), ledit deuxième élément d'étanchéité (106a) étant en prise avec ladite surface d'étanchéité (126a) dudit corps de soupape (108a) quand ladite pièce intérieure de soupape (102a) se trouve dans ladite position fermée, mais n'étant plus en prise avec ladite surface d'étanchéité (126a) dudit corps de soupape (108a) quand ladite pièce intérieure de soupape (1 02a) se trouve dans ladite position ouverte.

11. Soupape (100a) selon la revendication 1, ladite pièce intérieure de soupape (102a) comprenant au moins une indentation (136a) qui vient en butée contre une surface interne (138a) dudit corps de soupape (108a) quand ladite pièce intérieure de soupape (102a) est sollicitée dans ladite position fermée par lesdits moyens de ressort (110a).

12. Soupape (100a) selon la revendication 1, dans laquelle ledit corps de soupape (108a) comprend une poche (132a) et ladite pièce intérieure de soupape (102a) comprend un épaulement (134a), dans laquelle lesdits moyens de ressort (110a) sont disposés à l'intérieur de ladite poche (132a) dudit corps de soupape (108a) et coopèrent avec ledit épaulement (134a) de ladite pièce intérieure de soupape (1 02a).

13. Soupape (100a) selon la revendication 1, dans laquelle un matériau élastique (104b) est moulé par-dessus, ou co-injecté sur ladite pièce intérieure de soupape (102b).

14. Soupape (100a) selon la revendication 13, dans laquelle ledit matériau élastique (104b) est disposé sur ladite pièce intérieure de soupape (102a) à proximité dudit orifice (130a).
